# EUROPEAN PATENT SPECIFICATION

(11) **EP 4 351 617 B1**
(45) Date of publication and mention of the grant of the patent: **26.03.2025**
(21) Application number: 21742823.4
(22) Date of filing: 13.07.2021
(51) Int. Cl.: A61K 38/08, A61P 13/12, A61P 3/10, A61K 38/20

(54) **IL1RA DERIVED PEPTIDES FOR TREATMENT OF DIABETIC NEPHROPATHY**
VON IL1RA ABGELEITETE PEPTIDE ZUR BEHANDLUNG VON DIABETISCHER NEPHROPATHIE
PEPTIDES DÉRIVÉS D'IL1RA POUR LE TRAITEMENT D'UNE NÉPHROPATHIE DIABÉTIQUE

(43) Date of publication of application: 17.04.2024
(73) Proprietor: Serodus ApS, 2200 Copenhagen N (DK)
(72) Inventor: STEINESS, Eva, 2900 Hellerup (DK)
(74) Representative: COPA Copenhagen Patents
(86) International application number: PCT/EP2021/069450
(87) International publication number: WO 2023/284947

(56) References cited:
- US-A1- 2004 097 712
- US-A1- 2014 073 556
- US-A1- 2018 030 412
- DATABASE EMBASE [online] ELSEVIER SCIENCE PUBLISHERS, AMSTERDAM, NL; 7 July 2020 (2020-07-07), SALTI T ET AL: "Glucose Induces IL-1[alpha]-Dependent Inflammation and Extracellular Matrix Proteins Expression and Deposition in Renal Tubular Epithelial Cells in Diabetic Kidney Disease", Database accession no. EMB-632402232
- SALTI T ET AL: "Glucose Induces IL-1[alpha]-Dependent Inflammation and Extracellular Matrix Proteins Expression and Deposition in Renal Tubular Epithelial Cells in Diabetic Kidney Disease", FRONTIERS IN IMMUNOLOGY 20200707 FRONTIERS MEDIA S.A. CHE, vol. 11, 7 July 2020 (2020-07-07), XP055881737, ISSN: 1664-3224, DOI: 10.3389/fimmu.2020.01270
- DATABASE MEDLINE [online] US NATIONAL LIBRARY OF MEDICINE (NLM), BETHESDA, MD, US; 3 February 2014 (2014-02-03), KLEMENTIEV BORIS ET AL: "Anti-inflammatory properties of a novel peptide interleukin 1 receptor antagonist.", Database accession no. NLM24490798
- KLEMENTIEV BORIS ET AL: "Anti-inflammatory properties of a novel peptide interleukin 1 receptor antagonist.", JOURNAL OF NEUROINFLAMMATION 03 FEB 2014, vol. 11, 3 February 2014 (2014-02-03), pages 27, XP021176071, ISSN: 1742-2094, DOI: 10.1186/1742-2094-11-27
- DATABASE MEDLINE [online] US NATIONAL LIBRARY OF MEDICINE (NLM), BETHESDA, MD, US; 2020, SALTI TALAL ET AL: "Glucose Induces IL-1[alpha]-Dependent Inflammation and Extracellular Matrix Proteins Expression and Deposition in Renal Tubular Epithelial Cells in Diabetic Kidney Disease.", Database accession no. NLM32733443
- SALTI TALAL ET AL: "Glucose Induces IL-1[alpha]-Dependent Inflammation and Extracellular Matrix Proteins Expression and Deposition in Renal Tubular Epithelial Cells in Diabetic Kidney Disease.", FRONTIERS IN IMMUNOLOGY 2020, vol. 11, 2020, pages 1270, XP055881737, ISSN: 1664-3224, DOI: 10.3389/fimmu.2020.01270
- DATABASE MEDLINE [online] US NATIONAL LIBRARY OF MEDICINE (NLM), BETHESDA, MD, US; October 2005 (2005-10-01), LEE SANG-HO ET AL: "Genetics of diabetic nephropathy in type 2 DM: candidate gene analysis for the pathogenic role of inflammation.", Database accession no. NLM16174285
- LEE SANG-HO ET AL: "Genetics of diabetic nephropathy in type 2 DM: candidate gene analysis for the pathogenic role of inflammation.", NEPHROLOGY (CARLTON, VIC.) OCT 2005, vol. 10 Suppl, October 2005 (2005-10-01), pages S32 - S36, XP055881739, ISSN: 1320-5358, DOI: 10.1111/j.1440-1797.2005.00454.x
- SHAHZAD KHURRUM ET AL: "Nlrp3-inflammasome activation in non-myeloid-derived cells aggravates diabetic nephropathy", KIDNEY INTERNATIONAL, vol. 87, no. 1, 1 January 2015 (2015-01-01), GB, pages 74 - 84, XP055881761, ISSN: 0085-2538, DOI: 10.1038/ki.2014.271
- SALTI TALAL ET AL: "Glucose Induces IL-1[alpha]-Dependent Inflammation and Extracellular Matrix Proteins Expression and Deposition in Renal Tubular Epithelial Cells in Diabetic Kidney Disease", FRONTIERS IN IMMUNOLOGY, vol. 11, 1 January 2020 (2020-01-01), pages 1270, XP055881737, DOI: 10.3389/fimmu.2020.01270
- BORIS KLEMENTIEV ET AL: "Anti-inflammatory properties of a novel peptide interleukin 1 receptor antagonist", JOURNAL OF NEUROINFLAMMATION, BIOMED CENTRAL LTD., LONDON, GB, vol. 11, no. 1, 3 February 2014 (2014-02-03), pages 27, XP021176071, ISSN: 1742-2094, DOI: 10.1186/1742-2094-11-27
- CUCAK HELENA ET AL: "The IL-1 [beta] Receptor Antagonist SER140 Postpones the Onset of Diabetes in Female Nonobese Diabetic Mice", JOURNAL OF DIABETES RESEARCH, vol. 2016, 1 January 2016 (2016-01-01), pages 1 - 10, XP055882030, ISSN: 2314-6745, Retrieved from the Internet <URL:http://downloads.hindawi.com/journals/jdr/2016/7484601.pdf> DOI: 10.1155/2016/7484601
- SERODUS: "ANNUAL REPORT 2020", 1 January 2020 (2020-01-01), XP055882036, Retrieved from the Internet <URL:https://serodus.com/annual-reports/> [retrieved on 20220120]
- SERODUS: "SER140 - Serodus", 19 April 2021 (2021-04-19), XP055882038, Retrieved from the Internet <URL:https://web.archive.org/web/20210419192221/https://serodus.com/ser140/> [retrieved on 20220120]
- SERODUS: "PROSPECTUS (Norwegian: registrerings prospekt)", 1 March 2020 (2020-03-01), XP055882041, Retrieved from the Internet <URL:https://serodus.com/prospectus/> [retrieved on 20220120]

## Description

### Technical field

The present disclosure relates to peptides derived from IL-1R antagonist protein (IL1RA) for treatment of diabetic nephropathy.

### Background

Interleukin 1 (IL-1) is a general name for two distinct pro-inflammatory cytokines proteins, IL-1 alpha and IL-1 beta. IL-1 exerts its effects by binding to specific transmembrane receptors (IL-1R) on multiple cell types. The effects of IL-1 are counteracted by natural inhibitors such as soluble IL-1 receptors and IL-1R antagonist protein (IL1RA). IL1RA inhibits the effect of IL-1 by blocking its interaction with the cell surface receptors.

Therapeutic approaches for targeting IL-1 for anti-inflammatory purposes have been addressed in the art. These include administration of recombinant IL-1R antagonist protein, IL-1 trap fusion proteins, anti-IL-1 antibodies, anti-IL-1RI and soluble IL-1RI and II in experimental models of arthritis (reviewed in Gabay C et al. 2010).

Anakinra (US5075222) is a recombinantly produced protein that contains the *N*-terminal, methionylated, nonglycosylated version of human IL-1RA, which blocks the actions of IL-1 without any detectable agonist activity. Anakinra has been tested for the treatment of rheumatoid arthritis, adult-onset Still's disease, systemic onset juvenile idiopathic arthritis, osteoarthritis and type 2 diabetes mellitus. Anakinra is approved for treatment of rheumatoid arthritis; it is delivered as an injection concentrate with 100 mg in each dose; it is prepared from genetically modified E. coli using recombinant DNA technology; and it has a high molecular weight.

WO 2012/122985 and WO 2017/063657 discloses a 10-amino acid peptide derived from of IL1RA: SGRKSSKMQA (SEQ ID NO:1), and its use in treating various disorders. SEQ ID NO:1 has previously been shown to inhibit interleukin 1β-induced NF-kB signalling and macrophage secretion of TNFα, and hence is a potent inhibitor of inflammatory responses (Klementiev 2014).

Diabetic kidney disease (DKD), also known as diabetic nephropathy, has not historically been considered as an immune-mediated disease as metabolic and haemodynamic factors have been thought to be the main causes of renal injuries. However, recent studies support a role for the innate immune system in both the development and progression of DKD. Association with both systemic and local renal inflammation and an increased number of pro- and anti-inflammatory cytokines including IL-1 and IL-4 have been found in the kidney and some also in peripheral blood from patients with diabetic nephropathy in comparison to healthy control individuals (Hickey 2018, Araújo 2020, Murakoshi 2020).

The progressive non-infectious inflammation involves glomerular and tubular cells resulting in gradual loss of kidney function. The blood glucose and blood pressure shall be kept as normal as possible which will delay loss of kidney function. However, no specific treatment of this innate inflammation is developed, and the declining glomerular filtration and tubular function does not stop, and loss of renal function continue. At the end stage renal failure, the treatment will be chronic dialysis and in some cases kidney transplantation. Hence, interventions that prevent or halt the predestined inflammatory induced decline in all functions of nephrons focus on inhibition of the renal inflammation is needed.

Patients are diagnosed with diabetic nephropathy by measuring albumin in urine. Urine albumin is often measured in spot urine and corrected for creatinine in the same urine sample, ACR mg/g. The ACR are divided in three groups, normo-albuminuria ACR < 30 mg/g, micro-albuminuria ACR > 30 mg/g < 300 mg/g and macro-albuminuria ACR> 300 mg/g. There is clinical evidence that the higher ACR is the more advanced is the kidney damage.

Urine albumin and Kidney Injury Molecule-1 (KIM-1) are both biomarkers of kidney innate inflammation as seen in various autoimmune diseases which involve the kidneys as well as in other non-autoimmune diseases (Coca 2017, Cai 2019, Levey 2019). Human renal biopsies have confirmed the presence of inflammatory cells in both glomeruli, the tubuli and the interstitium at all stages of diabetic nephropathy (Kahn 2019, Calle 2021).

### Summary

It is shown herein that peptides derived from IL1RA according to the present disclosure significantly reduce urinary albumin corrected for creatinine (ACR) and the kidney-injuring molecule corrected for creatinine (KIM-1/CR). Urine albumin and KIM-1 are both biomarkers of kidney innate inflammation as seen in various diseases which involve the kidneys, including diabetic nephropathy.

As diabetic nephropathy has not historically been considered as an immune-mediated disease, there is a need for compounds and medicaments addressing the immune-mediated and renal inflammation aspects of diabetic nephropathy.

On the basis of the experimental data provided herein, peptides and compounds derived from IL4RA according to the present disclosure are likely to be able to prevent or halt the predestined inflammatory induced decline in various functions of nephrons of the kidney.

On the basis of the experimental data provided herein, peptides and compounds derived from IL4RA according to the present disclosure are likely to be able to inhibit renal inflammation which is observed in diabetic nephropathy.

The invention is set out in the appended set of claims.

### Description of Drawings

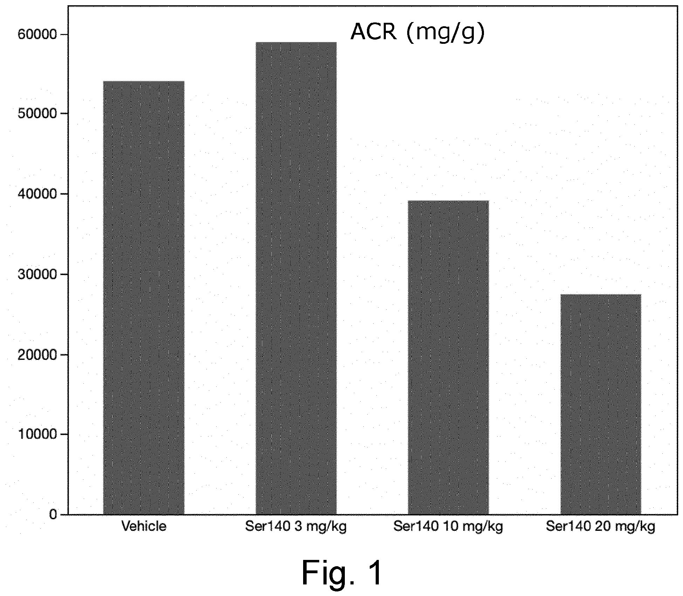

Figure 1 shows urinary albumin corrected for creatinine (ACR) in a urine sample after treatment with vehicle, 3 mg/kg SER140, 10 mg/kg SER140 and 20 mg/kg SER140 of Renin AAV Uni-nephrectomized db/db (Renin AAV UNx) mice for a period of 77 days. Figure 1 shows the medians of the different groups. See Example 1.

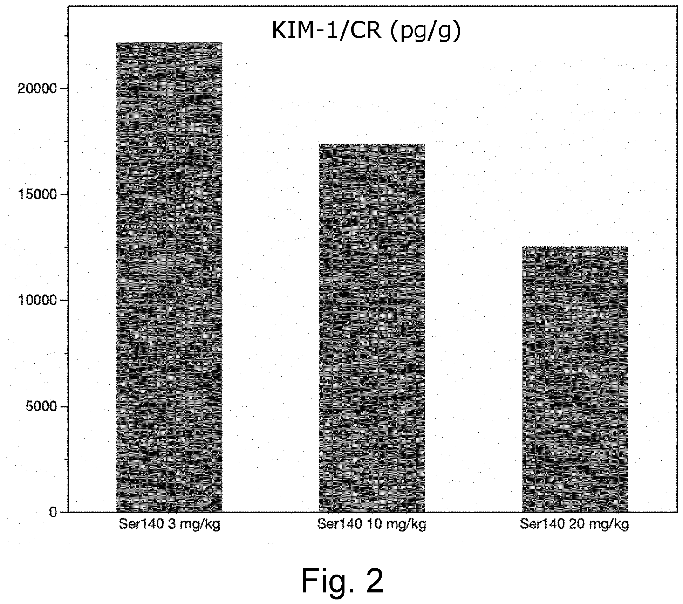

Figure 2 shows urinary KIM-1 corrected for creatinine (KIM-1/CR) in a urine sample after treatment with 3 mg/kg SER140, 10 mg/kg SER140 and 20mg/kg SER140 of Renin AAV Uni-nephrectomized (Renin AAV UNx) mice for a period of 77 days. Figure 2 shows the means of the different groups. See Example 1.

### Definitions and abbreviations

ACR: Albumin corrected for creatinine
DKD: Diabetic kidney disease.
IL1RA or IL1Ra: IL-1 receptor antagonist protein, may also be denoted IL-1 receptor antagonist herein.
IL-1: Interleukin 1.
IL1R1 or IL1R1: IL1 receptor type 1.
KIM-1: Kidney Injury Molecule-1
KIM-1/CR: KIM-1 corrected for creatinine
ReninAAV UNx mice: Renin AAV uninephrectomized db/db mice

The terms "diabetic nephropathy" and "diabetic kidney disease (DKD)" are used interchangeably to refer to the same disease. Diabetic nephropathy is a type of progressive kidney disease that may occur in individuals with diabetes mellitus. It affects individuals with type 1 and type 2 diabetes, and risk increases with the duration of the disease and other risk factors like high blood pressure and a family history of kidney disease.

The term "individual" refers to vertebrates, particular members of the mammalian species, preferably primates including humans. As used herein, 'subject' and 'individual' may be used interchangeably.

An "individual in need thereof" refers to an individual who may benefit from the present disclosure. In one embodiment, the individual in need thereof is a diseased individual. In one embodiment, the individual in need thereof is a diseased individual, in particular an individual with diabetes mellitus, type 1 or 2.

"Co-administering" or "co-administration" of compounds of the present disclosure and other medicaments, as used herein, refers to the administration of one or more compounds of the present disclosure and another medicament within a certain time period. The time period might be less than 72 hours, such as 48 hours, for example less than 24 hours, such as less than 12 hours, for example less than 6 hours, such as less than 3 hours. However, these terms also mean that the compound and a therapeutic composition can be administered together. Co-administration may be simultaneous, separately or sequentially.

An "effective amount" of a compound can be administered in one administration, or through multiple administrations of an amount that total an effective amount, for instance within a 24-hour period. It can be determined using standard clinical procedures for determining appropriate amounts and timing of administration. It is understood that the "effective amount" can be the result of empirical and/or individualized (case-by-case) determination on the part of the treating health care professional and/or individual.

"Sequence identity" as used herein is the number of amino acid residues which match exactly between two different sequences to be compared. If a variant is 90% identical to a sequence having ten amino acid residues, the variant can differ from the sequence by one amino acid substitution.

The terms "treating", "treatment" and "therapy" as used herein refer equally to curative therapy, prophylactic or preventative therapy and ameliorating or palliative therapy. The term includes an approach for obtaining beneficial or desired physiological results, which may be established clinically. For purposes of this disclosure, beneficial or desired clinical results include, but are not limited to, alleviation of symptoms, diminishment of extent of disease, stabilized (i.e., not worsening) condition, delay or slowing of progression or worsening of condition/symptoms, amelioration or palliation of the condition or symptoms, and remission (whether partial or total), whether detectable or undetectable. The term "palliation", and variations thereof, as used herein, means that the extent and/or undesirable manifestations of a physiological condition or symptom are lessened and/or time course of the progression is slowed or lengthened, as compared to not administering compositions of the present disclosure.

A "treatment effect" or "therapeutic effect" is manifested if there is a change in the condition being treated, as measured by the criteria constituting the definition of the terms "treating" and "treatment." There is a "change" in the condition being treated if there is at least 5% improvement, alternatively 10% improvement, alternatively at least 25%, alternatively at least 50%, such as at least 75%, alternatively at least 100% improvement. The change can be based on improvements in the severity of the treated condition in an individual, or on a difference in the frequency of improved conditions in populations of individuals with and without treatment with the compound, or with the compound in combination with a pharmaceutical composition of the present disclosure.

A treatment according to the disclosure may be prophylactic, ameliorating or curative.

The term "variant" means that the peptide sequence may be modified, for example by substitution of one or more of the amino acid residues. Both L-amino acids and D-amino acids may be used. Other modification may comprise derivatives such as esters, sugars, etc., for example methyl and acetyl esters, as well as polyethylene glycol modifications.

Furthermore, an amine group of the peptide may be converted to amides, wherein the acid part of the amide is a fatty acid.

The groups of conservative amino acids are as the following:
A, G (neutral, weakly hydrophobic),
Q, N, S, T (hydrophilic, non-charged)
E, D (hydrophilic, acidic)
H, K, R (hydrophilic, basic)
L, P, I, V, M, F, Y, W (hydrophobic, aromatic)
C (cross-link forming)

Conservative substitutions may be introduced in any position of a preferred predetermined peptide for use according to the disclosure. It may however also be desirable to introduce non-conservative substitutions, particularly, but not limited to, a non-conservative substitution in any one or more positions.

The peptide sequences of the present disclosure may be prepared by any conventional synthetic methods, recombinant DNA technologies, enzymatic cleavage of full-length proteins which the peptide sequences are derived from, or a combination of said methods.

Formulations of the compounds of the disclosure can be prepared by techniques known to the person skilled in the art. The formulations may contain pharmaceutically acceptable carriers and excipients including microspheres, liposomes, microcapsules, nanoparticles or the like. The active compound may be formulated as neutral or salt forms.

### Detailed description

According to an embodiment, the present disclosure concerns a compound for use in the treatment of diabetic nephropathy, wherein the compound comprises one or more peptides, each peptide consisting of the amino acid sequence SGRKSSKMQA (SEQ ID NO:1), or a variant thereof having SEQ ID NO:1 with one amino acid substitution.

According to an embodiment, the present disclosure concerns use of a compound for the manufacture of a medicament for treating diabetic nephropathy, wherein the compound comprises one or more peptides, each peptide consisting of the amino acid sequence SGRKSSKMQA (SEQ ID NO:1), or a variant thereof having SEQ ID NO:1 with one amino acid substitution.

In some embodiments of the present disclosure, the peptide binds to IL-1 receptor type 1 (ILR1), and/or interferes with the binding of IL-1 beta to IL1R1.

In some embodiments of the present disclosure, the variant of SEQ ID NO: 1 comprises one amino acid substitution.

In another embodiment of the present disclosure, the amino acid substitution(s) is a conservative amino acid substitution.

In some embodiments of the present disclosure, said one or more peptides consist of the amino acid sequence of SEQ ID NO:1, or a variant having 1 amino acid substitution.

In some embodiments of the present disclosure, said one or more peptides consist of a variant of SEQ ID NO:1 having 1 amino acid substitution.

In some embodiments of the present disclosure, said one or more peptides consist of the amino acid sequence of SEQ ID NO:1.

In another embodiment of the present disclosure, the C-terminal amino acid exists as the free carboxylic acid ("-OH"). In another embodiment, the C-terminal amino acid is an amidated derivative ("-NH¬2"). In another embodiment, the N-terminal amino acid comprises a free amino-group ("H-"). In another embodiment, the N-terminal amino acid is the acetylated derivative ("-Acetyl" or "COCH3").

In some embodiments of the present disclosure the compound for use is a multimer. In some embodiments of the present disclosure the compound for use is a multimer comprising at least two peptides, such as two or more peptides, according to the present disclosure.

In some embodiments of the present disclosure the compound for use is a multimer.

In some embodiments of the present disclosure the compound for use is a multimer comprising two or more peptides, each peptide consisting of SEQ ID NO: 1, or a variant thereof having SEQ ID NO:1 with one amino acid substitution.

In some embodiments of the present disclosure, the compound is a dimer, a trimer or a tetramer.

In some embodiments of the present disclosure, the compound for use is a dimer comprising two peptides, each peptide consisting of SEQ ID NO:1, or a variant thereof having SEQ ID NO:1 with one amino acid substitution.

In some embodiments of the present disclosure, the compound for use is a tetramer comprising four peptides, each peptide consisting of SEQ ID NO:1, or a variant thereof having SEQ ID NO:1 with one amino acid substitution.

In some embodiments, the compound is a dendrimer. In some embodiments, the dendrimer is a dimeric dendrimer. In some embodiments, the dendrimer is a tetrameric dendrimer.

Dendrimers are highly ordered, branched polymeric molecules. Typically, dendrimers are symmetric about the core, and often adopt a spherical three-dimensional morphology.

In some embodiments of the present disclosure, the compound for use is a dimeric dendrimer comprising two peptides, each peptide consisting of SEQ ID NO:1, or a variant thereof having SEQ ID NO:1 with one amino acid substitution.

In some embodiments of the present disclosure, the compound for use is a tetrameric dendrimer comprising four peptides, each peptide consisting of SEQ ID NO:1, or a variant thereof having SEQ ID NO:1 with one amino acid substitution.

In some embodiments of the present disclosure, the two or more peptides are linked via a peptide bond.

In some embodiments of the present disclosure, the two or more peptides are linked via a linker group.

In some embodiments of the present disclosure, the linker group is a lysine backbone, such as a lysine backbone comprising one or more lysine residues, such as a plurality of lysine residues.

In some embodiments of the present disclosure, the linker group comprises one or more lysine residues.

In some embodiments of the present disclosure, the compound for use is a dimeric dendrimer comprising two peptides, each peptide consisting of SEQ ID NO:1, or a variant thereof having SEQ ID NO:1 with one amino acid substitution, and a lysine backbone.

In some embodiments of the present disclosure, the compound for use is a tetrameric dendrimer comprising four peptides, each peptide consisting of SEQ ID NO:1, or a variant thereof having SEQ ID NO:1 with one amino acid substitution, and a lysine backbone.

In some embodiments of the present disclosure, the peptide is a tetrameric dendrimer consisting of four peptides, each peptide consisting of SEQ ID NO:1, and a lysine backbone.

In another embodiment of the present disclosure, the linker group is a polymer carrier, such as a protein carrier.

According to an embodiment, the present disclosure concerns a multimeric compound for use in the treatment of diabetic nephropathy, wherein the multimeric compound comprises at least two peptides, each peptide consisting of the amino acid sequence SGRKSSKMQA (SEQ ID NO:1), or a variant thereof having SEQ ID NO:1 with one amino acid substitution.

In some embodiments of the present disclosure, the multimeric compound is dendrimer.

In some embodiments of the present disclosure, the multimeric compound is a dimer, trimer or tetramer.

In some embodiments of the present disclosure, the multimeric compound is a dimer.

In some embodiments of the present disclosure, the multimeric compound is a tetramer.

In another embodiment of the present disclosure, the at least two peptides are identical.

According to an embodiment, the present disclosure concerns a multimeric compound for use in the treatment of diabetic nephropathy, wherein said multimeric compound comprises four peptides, each peptide consisting of the amino acid sequence SGRKSSKMQA (SEQ ID NO:1), or a variant thereof having SEQ ID NO:1 with one amino acid substitution.

In some embodiments of the present disclosure said four peptides are linked via a peptide bond.

In some embodiments of the present disclosure said four peptides are linked via a linker group.

In some embodiments of the present disclosure said linker group is a lysine backbone comprising one or more lysine residues, such as a plurality of lysine residues.

In some embodiments of the present disclosure the four peptides are identical.

In another embodiment of the present disclosure, the compound is formulated as a pharmaceutical composition.

In another embodiment of the present disclosure, the peptide or the compound decreases the urine albumin to creatinine ratio (ACR). In another embodiment, the peptide or the compound decreases the urine albumin to creatinine ratio (ACR) in a dose dependent manner.

In another embodiment of the present disclosure, the peptide or the compound decreases the urine level of the biomarker Kidney Injury Molecule-1 (KIM-1). In another embodiment, the peptide or the compound decreases the urine KIM-1 to creatinine ratio (KIM-1/CR). In another embodiment, the urine level of KIM-1 or the urine KIM-1 to creatinine ratio (KIM-1/CR) is decreased in a dose dependent manner.

In another embodiment of the present disclosure, the individual has diabetes mellitus, has ACR levels above 30, and/or has increased blood pressure. In another embodiment, the individual has normal blood pressure. In another embodiment, the individual has type I diabetes mellitus or type II diabetes mellitus.

In another embodiment of the present disclosure, the individual is treated with antidiabetics. In another embodiment, the individual is treated with antihypertensives.

In another embodiment of the present disclosure, the compound is administered in combination with an effective amount of an antidiabetic compound and/or an antihypertensive compound. In another embodiment, the antihypertensive compound is an ACE inhibitor or angiotensin receptor antagonist.

### Examples

### Example 1: Effect of IL1RA derived peptide om markers of diabetic nephropathy

### Materials and methods

### Animals

56 female mice (BKS.Cg-Dock7m +/+ Lepradb/db BLKS, 5 weeks of age) were transferred to the Gubra Research animal unit. The animals were group housed until
uni-nephrectomies, then single housed. Throughout the habituation and study period the animals were in a light-, temperature- and humidity-controlled room with free access to food and water. All animal experiments were conducted in accordance with Gubra bioethical guidelines, which are fully compliant to internationally accepted principles for the care and use of laboratory animals.

Animals were terminated on day 77; blood was taken for later analysis, body weight was recorded, and spot urine was collected.

### Compounds

SER140 was provided by Phlogo ApS, Copenhagen, Denmark (10mg/ml miliQ water diluted to the different dose formulations). SER140 is a dendrimer composed of four monomeric peptides each with the sequence SGRKSSKMQA (SEQ ID NO:1), and coupled to a lysine backbone. Vehicle was miliQ:PBS 1:5.

### In vivo procedures

After one week of acclimatization the mice received an IV injection of Renin adenosin-associated virus (AAV). One week later all mice were uni-nephrectomized. Two weeks after the Renin AAV iv injection the mice were randomized according to blood glucose to four groups: a vehicle group (n=14), a 3 mg/kg SER140 group, a 10 mg/kg SER140 and a 20 mg/kg SER140 group. The compound as well as the vehicle was administered subcutaneously once daily.

### Biochemical analysis

All blood samples were taken from the mice tail in heparinized tubes, plasma was separated and stored at minus 80 C until analysis. Non-fasting BG (blood glucose) was measured using BIOSEN c-line glucose meter (EKF-diagnostics, Germany), blood for HbA1c analysis was immediately suspended in Hemolyzing Reagent and stored until analysis using autoanalyzer Cobas C-111 with commercial kit (Roche Diagnostics, Germany), plasma urea using commercial kits (Roche Diagnostics).

Urine samples were stored at minus 80 C until analysis. Urine for measurement of albumin and creatinine were centrifuged at 2000 g for 2 min prior to analysis Urine albumin was measure using a commercial ELISA kit (Bethyl Laboratories, Inc.), according to the manufacturer's instructions, and urine creatinine was measured using a commercial kit (Roche Diagnostics) on the Cobas c 501 autoanalyzer, according to the manufacturer's instructions. Urinary KIM-1 was measured using a commercial ELISA kit (R&D Systems), according to the manufacturer's instructions.

### Results

The tested compound SER140 decreased ACR mg/g (Figure 1) and KIM-1/CR pg/g (Figure 2) significantly in a dose dependent manner.

There were no changes in HbA1c% between vehicle and SER140 treatment, indicating that the effect of SER140 on ACR and KIM-1 are not mediated via an effect on HbA1c.

### Discussion

ReninAAV UNx db/db mice is described as one of the best pharmacological models to describe development of diabetic kidney disease (DKD, diabetic nephropathy) and to elucidate compounds influence on the progression of the disease (Nguyen 2019, Sembach 2020). Recent studies indicate that immune system activation and imbalance of immune homeostasis play an important role in the development and progression of diabetic nephropathy.

It is well known that IL-1 cytokine consists of three different forms, the Il-1α, Il-1β, both IL-1 receptor agonists and IL-1Ra, a natural Il-1 receptor antagonist. All are ligands to the same IL-1R1 receptor. Both Il-1α and Il-1β are pro-inflammatory cytokines and are activating downstream while IL-1Ra binds to the IL-1R1 receptor without activating and thus act as a receptor antagonist and modulates a variety of II-1 related immune and inflammatory responses (Arend 2000, Dinarello 2012, Park 2015).

Urine albumin and KIM-1 are both biomarkers of kidney innate inflammation as seen in various autoimmune diseases which involve the kidneys. Human renal biopsies have confirmed the presence of inflammatory cells in both glomeruli, the tubuli and the interstitium at all stages of diabetic nephropathy (Kahn 2019, Calle 2021).

SER140 is a peptide derived from the human IL-1RA N-terminal domain, which is involved in interactions with IL-1R1. SER140 has been shown to inhibit receptor binding of IL-1β and therefore reduce the downstream activation of among other the macrophages liberation of TNFα (Klementiev 2014).

SER140 is shown herein to significantly reduced both urinary albumin, a biomarker of cellular damage of mainly glomerular endothelial and podocyte, and KIM-1, a biomarker of damage of the apical membrane of the proximal renal tubule. These biomarkers are undetectable in urine from patients with normal kidney but detectable is response to ischemic or toxic insults, including diabetes (Alderson 2016, Treacy 2019).

### Sequence overview

SEQ ID NO:1
   SGRKSSKMQA
SEQ ID NO:2 (IL1RA isoform 1) 
MEICRGLRSHLITLLLFLFHSETICRPSGRKSSKMQAFRIWDVNQKTFYLRNNQLVAGYLQGPNVNLEEKI
DVVPIEPHALFLGIHGGKMCLSCVKSGDETRLQLEAVNITDLSENRKQDKRFAFIRSDSGPTTSFESAACPG
WFLCTAMEADQPVSLTNMPDEGVMVTKFYFQEDE
SEQ ID NO:3 (IL1RA isoform 2) 
MALETICRPSGRKSSKMQAFRIWDVNQKTFYLRNNQLVAGYLQGPNVNLEEKIDVVPIEPHALFLGIHGG
KMCLSCVKSGDETRLQLEAVNITDLSENRKQDKRFAFIRSDSGPTTSFESAACPGWFLCTAMEADQPVSLT
NMPDEGVMVTKFYFQEDE
SEQ ID NO:4 (IL1RA isoform 3) 
MALADLYEEGGGGGGEGEDNADSKETICRPSGRKSSKMQAFRIWDVNQKTFYLRNNQLVAGYLQGPNV
NLEEKIDVVPIEPHALFLGIHGGKMCLSCVKSGDETRLQLEAVNITDLSENRKQDKRFAFIRSDSGPTTSFES
AACPGWFLCTAMEADQPVSLTNMPDEGVMVTKFYFQEDE
SEQ ID NO:5 (IL1RA isoform 4)

MQAFRIWDVNQKTFYLRNNQLVAGYLQGPNVNLEEKIDVVPIEPHALFLGIHGGKMCLSCVKSGDETRLQL
EAVNITDLSENRKQDKRFAFIRSDSGPTTSFESAACPGWFLCTAMEADQPVSLTNMPDEGVMVTKFYFQE
DE

### References

Andrew S Levey 1: Change in Albuminuria and GFR as End Points for Clinical Trials in Early Stages of CKD: A Scientific Workshop Sponsored by the National Kidney Foundation in Collaboration With the US Food and Drug Administration and European Medicines Agency. Am J Kidney Dis. 2020 Jan;75(1).
Araújo S L et al: Renal expression of cytokines and chemokines in diabetic nephropathy. BMC Nephrology volume 21, Article number: 308 (2020).
Boris Klementiev et al: Anti-inflammatory properties of a novel peptide interleukin 1 receptor antagonist. Journal of Neuroinflammation volume 11, Article number: 27 (2014).
Cem Gabay, Céline Lamacchia, Gaby Palmer: IL-1 pathways in inflammation and human diseases. Review Nat Rev Rheumatol. 2010 Apr;6(4):232-41.
Fatima Abid Khan, Syeda Sadia Fatima, Ghulam Mustafa Khan, Sana Shahid: Evaluation of kidney injury molecule-1 as a disease progression biomarker in diabetic nephropathy. Pak J Med Sci. Jul-Aug 2019;35(4):992-996. doi: 10.12669/pjms.35.4.154.
Fionnuala B Hickey, Finian Martin: Role of the Immune System in Diabetic Kidney Disease. Review Curr Diab Rep. 2018 Mar 12;18(4):20.
Frederikke E Sembach, Mette V Østergaard, Niels Vrang, Bo Feldt-Rasmussen, Keld Fosgerau, Jacob Jelsing, Lisbeth N Fink: Rodent models of diabetic kidney disease: human translatability and preclinical validity. Review Drug Discov Today. 2021 Jan;26(1):200-217.
Giulio Cavalli et al: Interleukin 1α: a comprehensive review on the role of IL-1α in the pathogenesis and treatment of autoimmune and inflammatory diseases. Review Autoimmun Rev. 2021 Mar;20(3).
Helen V. Alderson et al: The Associations of Blood Kidney Injury Molecule-1 and Neutrophil Gelatinase-Associated Lipocalin with Progression from CKD to ESRD. Clin J Am Soc Nephrol. 2016 Dec 7; 11(12): 2141-2149.
Isabel Nguyen, Arianne van Koppen, and Jaap A. Joles: Animal Models of Diabetic Kidney Disease.
Jieru Cai: Kidney injury molecule-1 expression predicts structural damage and outcome in histological acute tubular injury. Ren Fail. 2019 Nov;41(1):80-87.
Maki Murakoshi 1, Tomohito Gohda 1, Yusuke Suzuki 1: Circulating Tumor Necrosis Factor Receptors: A Potential Biomarker for the Progression of Diabetic Kidney Disease. Review Int J Mol Sci. 2020 Mar 13;21(6):1957.
Oliver Treacy, Nigel N. Brown, and Goce Dimeski: Biochemical evaluation of kidney disease. Transl Androl Urol. 2019 May; 8(Suppl 2): S214-S223.
Priscila Calle and Georgina Hotter: Macrophage Phenotype and Fibrosis in Diabetic Nephropathy. Int J Mol Sci. 2020 Apr; 21(8): 2806.
Steven G. Coca, Girish N. Nadkarni, Yuan Huang, Dennis G. Moledina, Veena Rao, Jane Zhang, Bart Ferket, Susan T. Crowley, Linda F. Fried and Chirag R. Parikh: Plasma Biomarkers and Kidney Function Decline in Early and Established Diabetic Kidney Disease. JASN September 2017, 28 (9) 2786-2793.
W. Arend and C. Guthridge: Biological role of interleukin 1 receptor antagonist isoforms. Ann Rheum Dis. 2000 Nov; 59(Suppl 1).

## Claims

1. A compound for use in the treatment of diabetic nephropathy, wherein said compound is a multimer comprising at least two peptides, wherein said peptides consist of the amino acid sequence SGRKSSKMQA (SEQ ID NO:1), or a variant thereof having SEQ ID NO:1 with one amino acid substitution.

2. The compound for use according to claim 1, wherein said variant thereof is SEQ ID NO:1 with one conservative amino acid substitution.

3. The compound for use according to any of the preceding claims, wherein said compound is a tetrameric dendrimer comprising four peptides, each peptide consisting of SEQ ID NO:1, or a variant thereof having SEQ ID NO:1 with one amino acid substitution.

4. The compound for use according to any of the preceding claims, wherein said compound is a tetrameric dendrimer comprising four peptides, each peptide consisting of SEQ ID NO:1, or a variant thereof having SEQ ID NO:1 with one amino acid substitution, and further comprising a lysine backbone.

## Patentansprüche

1. Verbindung zur Verwendung bei der Behandlung von diabetischer Nephropathie, wobei die Verbindung ein Multimer ist, das mindestens zwei Peptide umfasst, wobei die Peptide aus der Aminosäuresequenz SGRKSSKMQA (SEQ ID NO:1) oder einer Variante davon, die SEQ ID NO:1 mit einer Aminosäuresubstitution aufweist, bestehen.

2. Verbindung zur Verwendung nach Anspruch 1, wobei die Variante davon SEQ ID NO:1 mit einer konservativen Aminosäuresubstitution ist.

3. Verbindung zur Verwendung nach einem der vorstehenden Ansprüche, wobei die Verbindung ein tetrameres Dendrimer ist, das vier Peptide umfasst, wobei jedes Peptid aus SEQ ID NO:1 oder einer Variante davon, die SEQ ID NO:1 mit einer Aminosäuresubstitution aufweist, besteht.

4. Verbindung zur Verwendung nach einem der vorstehenden Ansprüche, wobei die Verbindung ein tetrameres Dendrimer ist, das vier Peptide umfasst, wobei jedes Peptid aus SEQ ID NO:1 oder einer Variante davon, die SEQ ID NO:1 mit einer Aminosäuresubstitution aufweist, besteht und weiter ein Lysin-Grundgerüst umfasst.

## Revendications

1. Composé destiné à être utilisé dans le traitement de la néphropathie diabétique, dans lequel ledit composé est un multimère comprenant au moins deux peptides, dans lequel lesdits peptides sont constitués de la séquence d'acides aminés SGRKSSKMQA (SEQ ID NO:1), ou d'une variante de celle-ci présentant SEQ ID NO:1 avec une substitution d'acide aminé.

2. Composé à utiliser selon la revendication 1, dans lequel ladite variante est SEQ ID NO:1 avec une substitution conservatrice d'acide aminé.

3. Composé à utiliser selon l'une quelconque des revendications précédentes, dans lequel ledit composé est un dendrimère tétramérique comprenant quatre peptides, chaque peptide étant constitué de SEQ ID NO:1, ou d'une variante de celle-ci présentant SEQ ID NO:1 avec une substitution d'acide aminé.

4. Composé à utiliser selon l'une quelconque des revendications précédentes, dans lequel ledit composé est un dendrimère tétramérique comprenant quatre peptides, chaque peptide étant constitué de SEQ ID NO:1, ou d'une variante de celle-ci présentant SEQ ID NO:1 avec une substitution d'acide aminé, et comprenant en outre un squelette de lysine.
